(19) Europäisches Patentamt / European Patent Office / Office européen des brevets

(11) **EP 4 036 210 A1**

(12) **EUROPEAN PATENT APPLICATION**
published in accordance with Art. 153(4) EPC

(43) Date of publication:
**03.08.2022   Bulletin 2022/31**

(21) Application number: **20869424.0**

(22) Date of filing: **28.08.2020**

(51) International Patent Classification (IPC):
*C12M 1/34* $^{(2006.01)}$       *G01N 21/17* $^{(2006.01)}$
*C12Q 1/06* $^{(2006.01)}$

(52) Cooperative Patent Classification (CPC):
**C12M 1/34; C12Q 1/06; G01N 21/17**

(86) International application number:
**PCT/JP2020/032726**

(87) International publication number:
**WO 2021/059869 (01.04.2021 Gazette 2021/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.09.2019   JP 2019176197**

(71) Applicant: **Kyocera Corporation
Kyoto-shi Kyoto 612-8501 (JP)**

(72) Inventor: **WATANABE, Masaya
Kyoto-shi, Kyoto 612-8501 (JP)**

(74) Representative: **TBK
Bavariaring 4-6
80336 München (DE)**

(54)    **CELL DETECTION DEVICE AND CELL DETECTION METHOD**

(57)    A cell detector and a cell detection method detect cell proliferation. A cell detector (100) includes a light emitter (10), a culture vessel (40) that accommodates a culture medium (70) containing a cell (60), a first slit member (20) including a first slit (90) and a first light shield (95), and at least one light receiver (30) that receives light emitted from the light emitter (10) and passing through the culture medium (70) and the first slit (90) in the first slit member (20) in an order of the culture medium (70) and the first slit (90). The first light shield (95) in the first slit member (20) blocks light (LS2) emitted from the light emitter (10) and scattered by the cell (60) in the culture vessel (40) when the cell (60) is at a position overlapping the first slit (90).

FIG. 1

## Description

### FIELD

The present disclosure relates to a cell detector and a cell detection method for detecting cell proliferation.

### BACKGROUND

In cell culture, the state of cells and the increase in cell number are visually observed using a phase-contrast microscope (refer to, for example, Patent Literatures 1 to 3). Another known technique uses a direct-contact complementary metal-oxide-semiconductor (CMOS) sensor to image cells for observation of their proliferation (refer to, for example, Patent Literature 4).

### CITATION LIST

### PATENT LITERATURE

Patent Literature 1: Japanese Unexamined Patent Application Publication No. 2019-106944
Patent Literature 2: Japanese Unexamined Patent Application Publication (Translation of PCT Application) No. 2018-515787
Patent Literature 3: WO 2016/142043
Patent Literature 4: Japanese Unexamined Patent Application Publication (Translation of PCT Application) No. 2015-528311

### BRIEF SUMMARY

### TECHNICAL PROBLEM

However, the known techniques described in Patent Literatures 1 to 3 involve lengthy visual observation of cells. Culture of a large number of cells with these techniques can place a large burden on the operator and increase the operation time. The observation using the CMOS sensor with the known technique in Patent Literature 4 is suitable for detailed observation of cells, such as observation of their shapes. However, the technique provides local information alone and is not suitable for determining, for example, the rough number of cells in the entire well. In addition, the techniques in Patent Literatures 1 to 4 each use expensive equipment and involve high facility costs.

The development in bioengineering may allow industrialized cell culture, which expects efficient culture of cells on a larger scale.

Inexpensive cell detectors and cell proliferation methods that can easily and efficiently detect cell proliferation are thus awaited.

### SOLUTION TO PROBLEM

A cell detector according to an embodiment of the present disclosure includes a light emitter, a culture vessel that accommodates a culture medium containing a cell, a first slit member including a first slit and a first light shield, and at least one light receiver that receives light emitted from the light emitter and passing through the culture medium and the first slit in an order of the culture medium and the first slit. The first light shield in the first slit member blocks light emitted from the light emitter and scattered by the cell in the culture vessel when the cell is at a position overlapping the first slit.

A cell detection method according to an embodiment of the present disclosure is a cell detection method implementable with the above cell detector. The method includes placing the culture medium and the cell in the culture vessel, culturing the cell, illuminating the culture medium containing the cell with light emitted from the light emitter, and obtaining an amount of light passing through the first slit member and received by the light receiver, or an amount of light passing through the first slit member and the second slit member and received by the light receiver.

### ADVANTAGEOUS EFFECTS

The cell detector according to an embodiment of the present disclosure includes the first slit member including the first slit and the first light shield. The first light shield in the first slit member blocks light emitted from the light emitter and scattered by the cell in the culture vessel. When the cell is included in a portion of the culture medium corresponding to the first slit in the first slit member, the intensity of the light received by the light receiver (the current value generated in response to the received light, or more specifically, the current value resulting from photoelectric conversion, expressed as $Ip1$) is lower than the intensity of the light received by the light receiver when no cell is included in the portion (the current value resulting from photoelectric conversion, expressed as $Ip0$), or in other words, $Ip1 < Ip0$. This is because no scattered light component is received by the light receiver when the cell is included in the portion. This structure allows reliable detection of the cell in the portion of the culture medium corresponding to the first slit. For the cell remaining stationary, the intensity of the light received by the light receiver decreases from $Ip0$ initially to $Ip1$ over time, and decreases further as the cell proliferates two- or three-dimensionally. This allows substantially accurate estimation of the number of cells included in the entire culture medium. For the cell moving, the probability of the cell included in the portion of the culture medium corresponding to the first slit may be measured per unit time, for example, per minute or per hour (presence time per unit time). In another example, the number of cells crossing the portion of the culture medium corresponding to the first slit may be measured per unit of time. Based on these measurements, the number of cells included in the entire culture medium can be estimated substantially accurately.

The cell detector according to an embodiment of the present disclosure includes the first slit member including a plurality of first slits. This structure allows more precise measurement by averaging the detected values for the plurality of first slits. This allows more accurate estimation of the number of cells included in the entire culture medium.

The cell detector according to an embodiment of the present disclosure includes the light receiver located between the bottom surface of the first slit member and the bottom surface of the culture vessel. The first slit member and the light receiver may be integrated into a detection substrate that is accommodated in the culture vessel. When the number of proliferated cells exceeds a predetermined number, the detection substrate can be removed from the culture vessel and disposed of.

The cell detector according to an embodiment of the present disclosure further includes a second slit member including a second slit and a second light shield between the first slit member and the light receiver. The scattered light is blocked by the first slit member and the second slit member. This allows more effective blocking of the scattered light. This structure allows reliable detection of the cell in the portion of the culture medium corresponding to the first slit and the second slit.

The cell detector and the cell detection method according to one or more embodiments of the present disclosure with the above structure can easily detect cell proliferation at low costs. In addition, the cell detector according to an embodiment can be mass-produced at low costs and thus be disposable. The disposable cell detector can prevent contaminants from entering the culture medium.

## BRIEF DESCRIPTION OF DRAWINGS

FIG. 1 is a cross-sectional view of a cell detector 100 according to an embodiment of the present disclosure.
FIG. 2 is a block diagram of the cell detector 100 according to the embodiment of the present disclosure.
FIG. 3 is a plan view of a first slit in the embodiment of the present disclosure.
FIG. 4 is a cross-sectional view of a cell detector 101 according to an embodiment of the present disclosure.
FIG. 5 is a block diagram of the cell detector 101 according to the embodiment of the present disclosure.
FIG. 6 is a flowchart showing a cell detection method according to an embodiment of the present disclosure.

## DETAILED DESCRIPTION

A cell detector according to one or more embodiments of the present disclosure will now be described with reference to the drawings.

Detector

First Embodiment

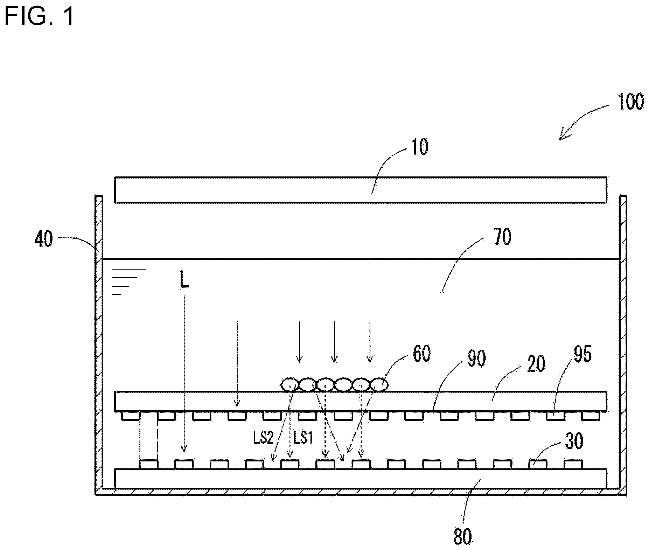

FIG. 1 is a schematic view of a cell detector 100 according to a first embodiment. The cell detector 100 according to the first embodiment includes a light emitter 10, a first slit member 20 including first slits 90 and first light shields 95, light receivers 30 that receive light emitted from the light emitter 10 and passes through a culture medium containing cells and the first slits 90 in the first slit member 20 in the stated order, and a culture vessel 40 for culturing cells.

A culture medium 70 (hereafter, also a culture substrate or cell culture medium) provides a growth environment for target cells in cell culture. The culture medium 70 is a source of nutrients such as a carbon source including glucose, a nitrogen source including peptone and ammonium sulfate, and inorganic salts including amino acids, vitamins, and phosphate. The culture medium 70 also provides a scaffold (platform) for cell proliferation. More specifically, the culture medium 70 may be a liquid medium or a solid medium. The liquid medium includes a liquid containing the above nutrients used for cell culture. The solid medium includes the liquid that is then solidified by adding agar or gelatin. The cell detector 100 according to the present embodiment may use a light-transmissive liquid medium allowing the cells to move in a plane (two-dimensionally) crossing the first slits 90. In some embodiments, the cell detector 100 may include a light-transmissive solid medium allowing the cells to slightly move two-dimensionally or to proliferate three-dimensionally above and overlapping the first slits 90.

The first light shields 95 in the first slit member 20 blocks light emitted from the light emitter 10 and scattered by cells 60 in the culture vessel 40. The scattered light includes a component reflected and scattered at the surfaces of the cells and a component transmitted through the cells and refracted at cell membranes. More specifically, scattered light corresponds to light incident on the main surface of the first slit member 20 substantially perpendicularly and is redirected by the cells. Arrows L (solid lines) in the figure indicate the path of light emitted from the light emitter 10, arrows LS1 (dashed lines) indicate the path of light transmitted straight through the cells 60, and arrows LS2 (dashed lines) indicate the path of light transmitted through and scattered by the cells 60.

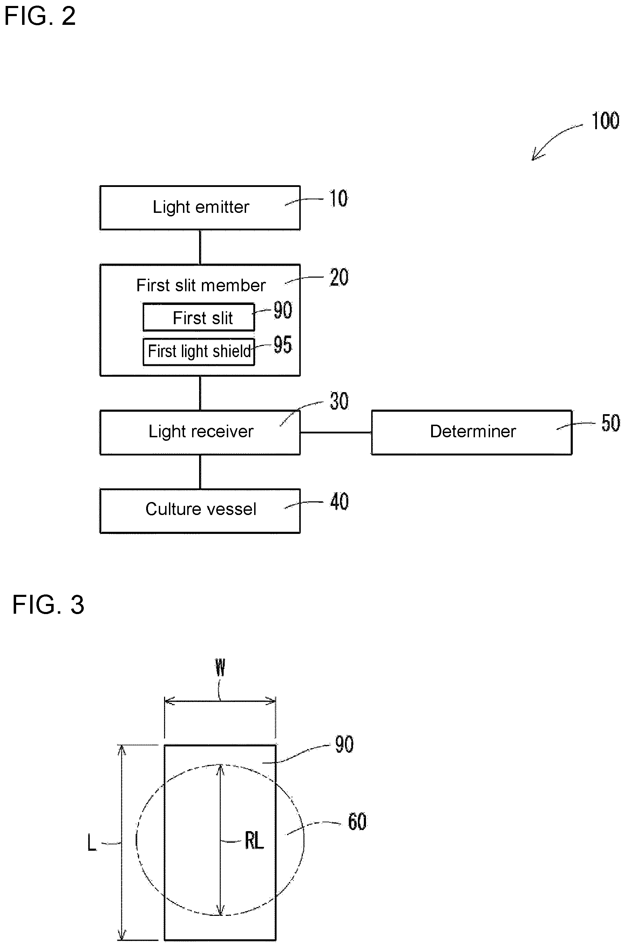

FIG. 2 is a block diagram of the cell detector 100 according to the first embodiment. The cell detector 100 according to the present embodiment includes the light emitter 10, the first slit member 20, the light receivers 30, the culture vessel 40, and a determiner 50. The determiner 50 determines that the cells 60 have undergone sufficient proliferation. The light emitter 10, the light receivers 30, and the determiner 50 are electrically connected to one another. The determiner 50 may be implemented with, for example, a central processing unit (CPU).

The determiner 50 may be eliminated in the present embodiment when the detection results can be output from the light receivers 30 to an external device. For example, the detection results may be displayed on an image display device (a display) included in an external personal computer. The electrical connection between the light receivers 30 and the determiner 50 may be a wired connection using a signal cable or a wireless connection using a transmitter and receiver and an antenna.

The light emitter 10 illuminates the cells 60 in the culture vessel 40 with light. The light emitter 10 may be, for example, a light-emitting device such as a light-emitting diode (LED), an electroluminescence device (EL device), a fluorescent lamp, or a laser light emitter such as a semiconductor laser element. The light emitter 10 faces the light receivers 30 with the first slit member 20 between them. The first slits 90 in the first slit member 20 and the light receivers 30 may overlap each other in a plan view. In this case, the light receivers 30 can receive light passing through the first slits 90 with high sensitivity. In addition, the difference (Ip0 - Ip1) between the intensity of the light received by the light receivers 30 when the cells 60 are included in portions of the culture medium 70 corresponding to the first slits 90 in the first slit member 20 (a current value resulting from photoelectric conversion, expressed as Ip1) and the intensity of the light received by the light receivers 30 when no cells 60 are included in the portions (a current value resulting from photoelectric conversion, expressed as Ip0) can be greater.

The first slit member 20 causes light emitted from the light emitter 10 and scattered by the cells 60 in the culture vessel 40 to avoid reaching the light receivers 30. The first light shields 95 in the first slit member 20 may be formed from, for example, a light-shielding material including a black metal layer such as a chromium (Cr) layer or a black resin layer (black matrix) located in a portion of the lower surface (the surface facing the light receivers 30) of a transparent substrate excluding the first slits 90. Examples of the transparent substrate include a glass substrate and a plastic substrate.

The first slit member 20 may include one first slit 90 alone or multiple first slits 90. For the first slit member 20 including one first slit 90, the first slit 90 may be, for example, cross-shaped, spiral-shaped, lattice-shaped, circular, elliptical, triangular, square, or rectangular in a plan view. The first slit 90 may have any shape as appropriate for the shape of the culture vessel 40, and the shape, size, motility including movement velocity, and the proliferation characteristics of the cells 60. For the first slit member 20 including multiple first slits 90, the first slits 90 may be, for example, rectangular in a plan view. FIG. 3 shows an example shape of the first slit 90.

As shown in FIG. 3, the rectangular first slit 90 may have a dimension in the width (W) direction smaller than the diameter of the cell 60 and a dimension in the length (L) direction, which is orthogonal to the width direction, larger than the length of the cell 60 (diameter RL) in the length direction. In this case, the scattered light LS2 (shown in FIG. 2) transmitted through, refracted, and scattered (redirected) by the cells 60 deviates from the path to each light receiver 30 in the length direction in a plan view while traveling toward the light receivers 30. This prevents scattered light from passing through the first slits 90 in the first slit member 20 and being received by the light receivers 30. For example, the rectangular first slit 90 may have a dimension of 7 to 25 $\mu$m in the width direction and a dimension of 25 to 1,000 $\mu$m in the length direction. More specifically, the first slit 90 may have a dimension of 25 to 50 $\mu$m in the length direction. The cells 60 may typically have curved peripheries, such as circular or elliptical peripheries in a plan view. The cell is circular in the example of FIG. 3. However, the cells 60 may have any shape other than a circle.

The cells 60 may be of any type, including animal cells, plant cells, yeast cells, or bacterial cells. Animal cells include muscle cells, visceral cells such as the liver, blood cells such as lymphocytes, monocytes, and granulocytes, nerve cells, immune cells, and induced pluripotent stem (iPS) cells. These cells may be tissue-derived primary cells or may be subcultured cells. iPS cells have pluripotency and the self-renewal ability. iPS cells are produced by introducing several types of genes into somatic cells, such as skin cells of a human, and culturing the cells to be pluripotent cells that can differentiate into cells of various tissues and organs, similarly to embryonic stem (ES) cells. The self-renewal ability allows iPS cells to retain pluripotency after division and proliferation, thus achieving substantially unlimited proliferation. The cell detector 100 according to the present embodiment can be used to manage the number of proliferated iPS cells with high proliferative ability. The cells 60 may be prokaryotic cells such as an Escherichia coli cell, or eukaryotic cells such as animal cells or plant cells. The cells 60 may be, for example, normal cells, abnormal cells such as tumor cells, or artificially created cells such as transgenic cells. The cells 60 may also be cultured as part of a living tissue. The cell culture may be adherent cell culture or suspension cell culture.

The first light shields 95 in the first slit member 20 may be formed from a light-shielding material in a portion of the upper surface (the surface facing the light emitter 10) of the first slit member 20 excluding the first slits 90. The light-shielding material may be, but not limited to, a black metal or a black resin such as a black resist.

The light receivers 30 receive light passing through the first slits 90 in the first slit member 20 without being transmitted through the cells 60 and light traveling straight after transmitted through the cells 60. Each light receiver 30 may include, for example, a light receiving element such as a silicon photodiode. Photodiodes can be mass-produced at lower costs using thin-film technologies including thin-film transistors.

The first slit member 20 is located under the cells 60 and the light receivers 30 are located directly below the first slit member 20. This structure allows detection of the scattering intensity of light scattered by the cells 60 using a difference in the light detection intensity, allowing detection of the cells 60 included in the portions of the culture medium 70 corresponding to the first slits 90. Formula 1 represents the amount of light $I_1$ received by the light receivers 30 when no cells 60 are included in the portions of the culture medium 70 corresponding to the first slits 90. Formula 2 represents the amount of light $I_1'$ received by the light receivers 30 when the cells 60 are included in the portions of the culture medium 70 corresponding to the first slits 90.

$$I_0 - B = I_1 \qquad (1)$$

$$I_0 - (B + A) - SL = I_1' \qquad (2)$$

In these formulas, $I_0$ is the amount of light emitted from the light emitter 10, A is the amount of light absorbed by the cells 60, B is the amount of light absorbed by the culture medium 70, SL is the amount of light scattered by the cells 60, and $I_1$ and $I_1'$ are the amount of light received by the light receivers 30. The term $I_0$ - (B + A) indicates the amount of light transmitted through the cell 60 and traveling straight without being scattered.

For the component transmitted through the cells 60 and traveling straight, the amount of light emitted from the light emitter 10 does not decrease substantially, except for the amount of light B absorbed by the culture medium 70. This is because the cells 60 have a high transmittance and thus A is a low value. However, the cells 60 each have an inherent refractive index, and light transmitted through the cells 60 are scattered. The light receivers 30 receive no scattered light. The amount of light SL thus becomes larger as the number of cells 60 increases, and the amount of light $I_1'$ received by the light receiver 30 decreases. The use of multiple first slits 90 allows more precise detection.

In other words, when proliferation of the cells 60 is insufficient in the early stage of cell culture, a small number of cells 60 are contained in the culture vessel 40, causing a slight decrease of light resulting from scattering. Light emitted from the light emitter 10 is thus received by the light receivers 30 with almost no scattering. After sufficient proliferation of the cells 60, a large number of cells 60 are densely contained in the culture vessel 40, thus scattering most of the light emitted from the light emitter 10 transmitted through the cells 60. A small amount of light is received by the light receiver 30. For the cells 60 that have undergone sufficient proliferation in the culture vessel 40, the amount of light received by the light receivers 30 corresponding to the number of proliferated cells 60 is measured in advance and stored as a predetermined amount into a storage table in, for example, a memory device. In response to an amount of light received by the light receivers 30 being smaller than or equal to the predetermined amount (greater than or equal to a predetermined number of proliferated cells), the cells 60 are determined to have proliferated to the predetermined number or greater. The predetermined number may be the number of cells per unit area, for example, per 1 mm$^2$ or per 1 cm$^2$. In some embodiments, the predetermined number may be the number of cells per unit volume, for example, per 1 mm$^3$ or per 1 cm$^3$. For example, the predetermined number may be about 1,000 to 100,000 per unit area or 100 to 100,000 per unit volume.

The determiner 50 determines that the cells 60 have proliferated to the predetermined number or greater in response to an amount of the light received by the light receivers 30 being smaller than or equal to the predetermined amount. The predetermined amount may be an amount determined in advance. For example, the predetermined amount may be the amount of light received by the light receivers 30 without being scattered by the cells 60 when the cells 60 have proliferated on the entire bottom surface of the culture vessel 40 or on the entire upper surface of the first slit member 20. The predetermined number may be a number determined in advance. For example, the predetermined number may be the number of cells 60 densely covering the entire bottom surface of the culture vessel 40 or the entire upper surface of the first slit member 20 for which the culture medium is to be replaced. The sufficient proliferation of the cells 60 can be determined by determining whether the cells 60 are dense in the culture vessel 40 and the proliferation rate is decreased (whether the cells 60 have reached confluence).

The structure according to a modification of the present embodiment may include as many light receivers 30 as the first slits 90. Each light receiver 30 may have dimensions in the width and length directions substantially equal to the dimensions of the first slit 90 in the width and length directions. Each light receiver 30 may be located at substantially the same position as the corresponding first slit 90 in the width and length directions in a plan view. The light receivers 30 each have an upper surface at a distance of 50 to 1,000 μm from the bottom surface of the first slit member 20. This structure allows light scattered by the cells 60 and passing through the first slits 90 in the first slit member 20 to travel without reaching the light receivers 30. This reliably prevents the light receivers 30 from receiving the scattered light. This structure can thus detect proliferated cells more accurately. The multiple light receivers 30 may be located on the upper surface of a base 80 (shown in FIG. 2) formed from a glass substrate or a plastic substrate.

The culture vessel 40 may include, for example, a Petri dish, a flask, and a microwell plate. The culture vessel 40 may have any shape or size, but may typically have one or more spaces suitable for proliferating the cells 60. For example, a Petri dish may have a width or a diameter of several centimeters to several tens of centimeters and a height of several millimeters to several centimeters. A flask may have a width or a diameter of several centimeters to several tens of centimeters and a height of five to several tens of centimeters. A microwell plate may have a width or a diameter of several centimeters to several tens of centimeters and a height of 0.5 to several centimeters. The culture vessel 40 is formed from an optically transparent material, such as a plastic material or a glass material to be observable from outside. The microwell plate has wells each in the shape of, for example, a circle, a rectangle such as a square, or a polygon such as a pentagon or a hexagon. The circular well is suitable for isometric proliferation of cells, allowing effective proliferation of the cells 60. The hexagonal well is suitable for the closest arrangement of wells, effectively reducing the size of the microwell plate. The culture vessel 40 accommodates the cells 60 and the culture medium 70. More specifically, the culture vessel 40 may be a commercially available cell culture vessel, such as a cell culture plate, a cell culture flask, or a cell culture dish. These cell culture vessels typically include lids and are formed from a transparent resin. The culture vessel 40 may include multiple cylindrical containers for accommodating the culture medium 70. The culture vessel 40 may be first attached to the cell detector 100 without the cells 60 and the culture medium 70, and then receive the cells 60 and the culture medium 70. In some embodiments, the culture vessel 40 accommodating the cells 60 and the culture medium 70 may be attached to the cell detector 100. The culture vessel 40 can be removed from the cell detector 100 once the cells 60 have undergone sufficient proliferation. After the cells 60 and the culture medium 70 are collected, the culture vessel 40 may be washed, sterilized, and reattached to the cell detector 100 for another use.

The light receivers 30 may be located below the bottom surface of the first slit member 20 and directly below the culture vessel 40. In this case, the cells 60 are cultured in the culture vessel 40 accommodating the culture medium 70. The cells 60 may adhere to the upper surface of the first slit member 20 or may be suspended in the culture medium 70. This structure can avoid adherence of the cells 60 and the culture medium 70 to the light receivers 30. The light receivers 30 are thus reusable without being washed and sterilized.

The first slit member 20 and the light receivers 30 may be located directly below the culture vessel 40. In this case, the cells 60 are cultured in the culture vessel 40 accommodating the culture medium 70. The cells 60 may adhere to the bottom of the culture vessel 40 or may be suspended in the culture medium 70. This structure can avoid adherence of the cells 60 and the culture medium 70 to the first slit member 20 and the light receivers 30. The first slit member 20 and the light receivers 30 are thus reusable without being washed and sterilized for multiple culture vessels 40.

The first slit member 20 and the light receivers 30 may be located directly above the bottom surface of the culture vessel 40. For example, the light receivers 30 may be located between the bottom surface of the first slit member 20 and the bottom surface of the culture vessel 40. In this case, the first slit member 20 and the light receivers 30 may be located in the culture medium 70. The cells 60 may adhere to the upper surface of the first slit member 20 or may be suspended in the culture medium 70. The first slit member 20 and the light receivers 30 may be disposable products to save the burden of washing and removing the cells 60 and the culture medium 70 and sterilizing the first slit member 20 and the light receivers 30.

The culture medium 70 may be any commercially available cell culture medium and is selected as appropriate for the cells 60 to be used. The culture medium 70 may be, for example, a Dulbecco's modified Eagle's medium when mammalian cells are to be cultured. The culture medium 70 may further contain additional components used for culturing the cells 60, such as bovine serum albumin, growth factors, amino acids, and antibiotics.

Cell culture typically involves medium replacement or subculturing every 2 to 4 days. Once the cells 60 have undergone sufficient proliferation in the culture vessel 40, the cells 60 and the culture medium 70 are collected to replace the culture medium or subculture. The collected cells 60 and the culture medium 70 may be used in subsequent experiments.

In a modification of the present embodiment, the cell detector 100 may include a light emitter 10, a culture vessel 40 accommodating a culture medium 70 containing cells 60, a first slit member 20 including first slits 90, and light receivers 30 that receive light emitted from the light emitter 10 and passes through the culture medium 70 and the first slits 90 in the stated order. The first slit member 20 may cause light emitted from the light emitter 10 and scattered by the cells 60 in the culture vessel 40 to avoid reaching the light receivers 30.

Second Embodiment

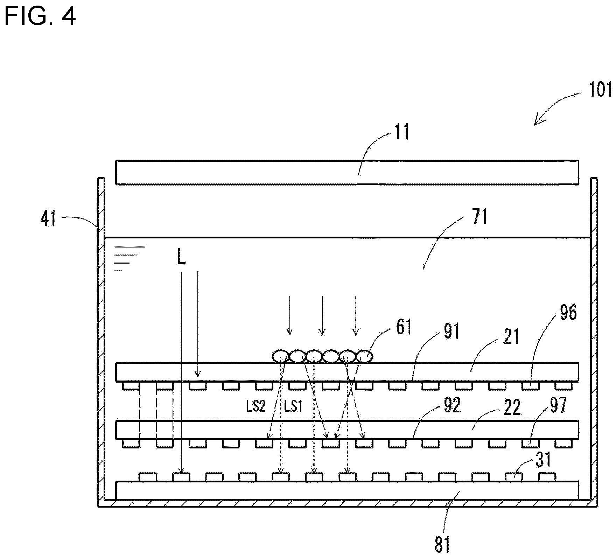

FIG. 4 is a schematic view of a cell detector 101 according to a second embodiment. The cell detector 101 according to the present embodiment includes a light emitter 11, a first slit member 21 including first slits 91 and first light shields 96, light receivers 31 that receive light emitted from the light emitter 11 and passing through the first slits 91 in the first slit member 21, and a culture vessel 41 for culturing cells.

The first light shields 96 in the first slit member

21 blocks light emitted from the light emitter 11 and scattered by the cells 61 in the culture vessel 41. The cell detector 101 further includes a second slit member 22 between the first slit member 21 and the light receivers 31. The second slit member 22 includes second slits 92 and second light shields 97.

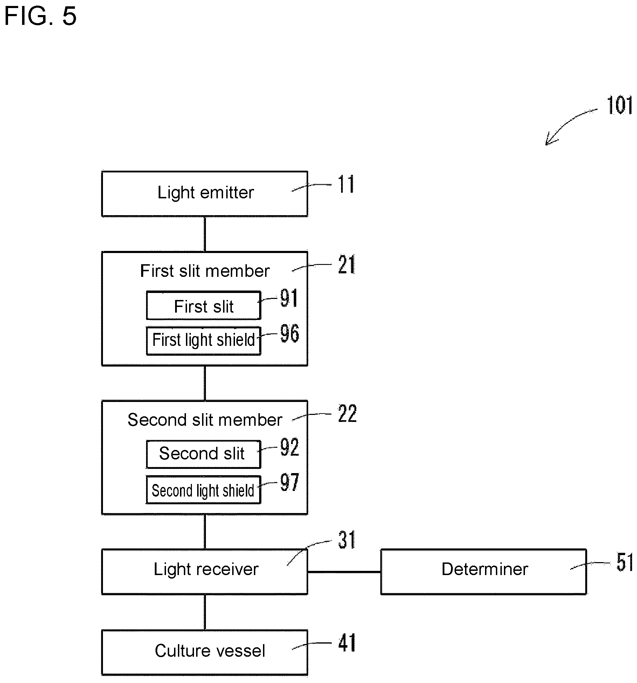

FIG. 5 is a block diagram of the cell detector 101 according to the second embodiment. In the present embodiment, the light emitter 11, the first slit member 21, the light receivers 31, the culture vessel 41, a determiner 51, the cells 61, the culture medium 71, the first slits 91, and the first light shields 96 are the same as those in the first embodiment unless otherwise specified and will not be described repeatedly.

The second slit member 22 blocks light passing through the first slits 91 in the first slit member 21 and scattered by the cells 61 and thus causes the scattered light to avoid reaching the light receivers 31. The second slit member 22 may have the same shape as the first slit member 21 and may be located at substantially the same position as the first slit member 21 in the width and length directions. The first slits 91 in the first slit member 21 and the second slits 92 in the second slit member 22 may substantially overlap each other in a plan view. Light emitted from the light emitter 11 and passing through the first slits 91 without being transmitted though the cells 61 may substantially entirely pass through the second slits 92. This structure increases the rate of decrease in the amount of light received by the light receivers 31 caused by the scattered light. This allows more accurate detection of the proliferation of the cells 61.

The second slit member 22 may have its upper surface at a distance from the bottom surface of the first slit member 21 to more effectively block the scattered light. The second slit member 22 may have the upper surface at a distance of 1 to 100 $\mu$m from the bottom surface of the first slit member 21. This structure allows the second slit member 22 to sufficiently block the scattered light passing through the first slits 91 in the first slit member 21, preventing the scattered light passing through the first slits 91 in the first slit member 21 from passing through the second slits 92 in the second slit member 22 and reaching the light receivers 31.

The second slits 92 each may have a shape similar to the first slit 91, and may have the same or a different dimension as or from the first slit 91. For example, when the distance between the bottom surface of the first slit member 21 and the upper surface of the second slit member 22 (distance D12) is shorter, the dimension of the second slit 92 may be the same as the dimension of the first slit 91. When the distance D12 is longer, the dimension of the second slit 92 may be larger than the dimension of the first slit 91. When the distance D12 is longer, light scattered by the cells 61 may diffuse over a larger area, and the culture medium 71 within the distance D12 absorbs more light, thus possibly lowering the sensitivity of the light receiving elements. When, for example, the distance D12 is shorter than or equal to the dimension of one cell 61 (about 10 to 50 $\mu$m), the second slit 92 may have the same dimension as the first slit 91. When the distance D12 exceeds the dimension of one cell 61, the second slit 92 may have a larger dimension than the first slit 91. In this case, the second slit 92 may have an opening area greater than one time and less than or equal to two times the opening area of the first slit 91.

The second light shields 97 in the second slit member 22 may be formed from, for example, a light-shielding material including a black metal layer such as a chromium (Cr) layer or a black resin layer (black matrix) located in a portion of the lower surface (the surface facing the light receivers 31) of a transparent substrate excluding the second slits 92. Examples of the transparent substrate include a glass substrate and a plastic substrate. The light shields 97 may be formed from a light-shielding material located in a portion of the upper surface (the surface facing the light emitter 11) of the second slit member 22 excluding the second slits 92.

The first slit member 21, the second slit member 22, and the light receivers 31 may be located directly below the culture vessel 41. In this case, the cells 61 are cultured in the culture vessel 41 accommodating the culture medium 71. The cells 61 may adhere to the bottom of the culture vessel 41 or may be suspended in the culture medium 71. This structure avoids adherence of the cells 61 and the culture medium 71 to the first slit member 21, the second slit member 22, and the light receivers 31. The first slit member 21, the second slit member 22, and the light receivers 31 are thus reusable without being washed and sterilized for multiple culture vessels 41.

The light receivers 31 receive light passing through the first slits 91 in the first slit member 21 and through the second slits 92 in the second slit member 22 without being transmitted through the cells 61 and light traveling straight after transmitted through the cells 61. Each light receiver 31 may include, for example, a light receiving element such as a silicon photodiode.

The light receiver 31 may have dimensions in the width and length directions larger than those of the second slit 92 in the second slit member 22. Light passing through the first slits 91 in the first slit member 21 is sufficiently blocked by the second slit member 22. The light receivers 31 may include multiple light receiving elements. For example, the multiple light receiving elements are arranged to cover the bottom surface of the culture vessel 41. As in the structure in FIG. 2, the light receivers 31 may be located on the upper surface of a base 81 formed from a glass substrate or a plastic substrate.

The light receivers 31 may be located between the bottom surface of the second slit member 22 and the bottom surface of the culture vessel 41. In this case, the first slit member 21, the second slit member 22, and the light receivers 31 may be located in the culture medium 71. The cells 61 may adhere to the upper surface of the first slit member 21 or may be suspended in the culture medium 71. The first slit member 21, the second slit member 22, and the light receivers 31 may be disposable products to save the burden of washing and removing the cells 61 and the culture medium 71 and sterilizing the first slit member 21, the second slit member 22, and the light receivers 31.

The light receivers 31 may be located below the bottom surface of the second slit member 22 and directly below the culture vessel 41. In this case, the first slit member 21 and the second slit member 22 may be located in the culture medium 71. The cells 61 may adhere to the upper surface of the first slit member 21 or may be suspended in the culture medium 71. This structure avoids adherence of the cells 61 and the culture medium 71 to the light receivers 31. The light receivers 31 are thus reusable without being washed and sterilized. The first slit member 21 and the second slit member 22 may be disposable products to save the burden of washing and removing the cells 61 and the culture medium 71 and sterilizing the first slit member 21 and the second slit member 22.

The first slit member 21 is located under the cells 61, the second slit member 22 is located directly below the first slit member 21, and the light receivers 31 are located directly below the second slit member 22. This structure allows detection of the scattering intensity of scattered light as a difference in the light detection intensity, allowing detection of the cells 61. Detection Method

Third Embodiment

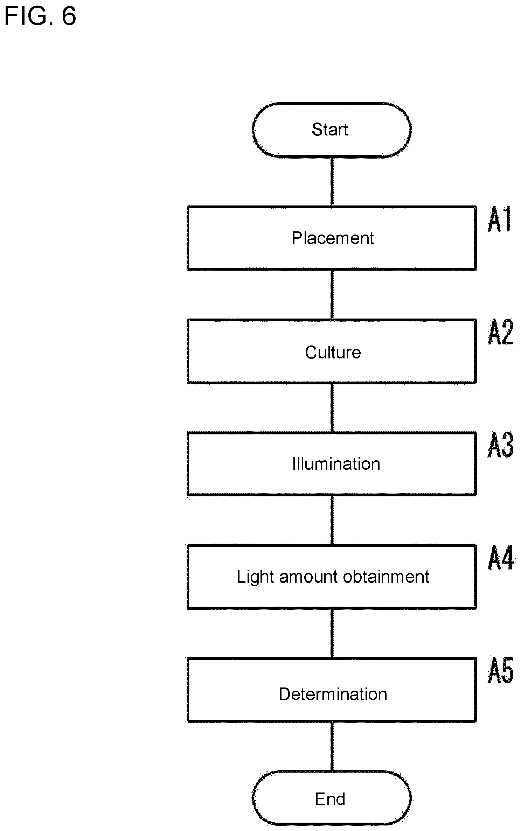

FIG. 6 is a flowchart of a cell detection method according to a third embodiment. The cell detection method according to the present embodiment is implementable with the cell detector according to the embodiments of present invention. The method includes placing the culture medium and the cells in the culture vessel (placement A1), culturing the cells (culture A2), illuminating the culture medium containing the cells with light emitted from the light emitter (illumination A3), and obtaining an amount of light passing through the first slit member and received by the light receiver, or an amount of light passing through the first slit member and the second slit member and received by the light receiver (light amount obtainment A4).

Placement A1 includes placing the culture medium and the cells in the culture vessel. The culture medium and the cells may be placed simultaneously. More specifically, the culture medium mixed with the cells may be placed in the culture vessel. After the culture medium is placed in the culture vessel, a small amount of culture medium containing cells may be placed further. The culture medium may be selected as appropriate for the type of the cells.

Culture A2 includes culturing the cells. The conditions for culturing such as the temperature, duration, and atmosphere may be selected as appropriate for the cells and the culture medium.

Illumination A3 includes illuminating the cells in the culture medium with light emitted from the light emitter. Illumination A3 is performed, for example, after culturing the cells for a period predetermined for the type and the number of cells and the type and the amount of culture medium.

Light amount obtainment A4 includes obtaining an amount of light received by the light receivers. As the cells proliferate, the amount of light transmitted through and scattered by the cells increases. The amount of light received by the light receivers is thus decreased.

In the present embodiment, the method may further include, after light amount obtainment A4, determining whether the cells have proliferated to a predetermined number or greater in response to an amount of the light received by the light receivers being smaller than or equal to a predetermined amount (determination A5). In determination A5, the amount of light received by the light receivers when the cells after sufficient proliferation are illuminated with light may be measured in advance as appropriate for the types of the cells and the culture medium, the number of the cells and the amount of the culture medium placed in the culture vessel, and the type of the culture vessel. In response to the cells being dense in the culture vessel and the proliferation rate being decreased in accordance with the types of the cells and the culture vessel or other factors, the cells may be determined to have proliferated to the predetermined number or greater. In determination A5, the determination may be repeated periodically, for example, every minute, every hour, or every day, before the amount of light received by the light receivers reduces to smaller than or equal to the predetermined amount.

As described in the above embodiments of the present invention, for example, the light emitter is located above the culture vessel accommodating the culture medium containing the cells, the light receivers are located below and face the light emitters with the culture vessel in between, and the first slit member including the first slits and the first light shields is located inside or outside the culture vessel. The structure may include multiple sets of the light emitter, the first slit member, and the light receivers. The light emitter may be located laterally to the culture vessel accommodating the culture medium containing the cells, the light receivers may be located laterally and face the light emitters with the culture vessel in between, and the first slit member including the first slits and the first light shields may be located inside or outside the culture vessel. This structure may include multiple sets of the light emitter, the first slit member, and the light receivers. The above structures allow more accurate estimation of the number of cells.

Although the embodiments of the present disclosure are described above specifically, the present disclosure is not limited to the above embodiments, but may be changed and modified variously without departing from the spirit and scope of the present disclosure. The components described in the above embodiments may be entirely or partially combined as appropriate unless any contradiction arises.

Reference Signs List

10, 11 light emitter
20, 21 first slit member
22 second slit member
30, 31 light receiver
40, 41 culture vessel
50, 51 determiner
60, 61 cell
70, 71 culture medium
80 base
90, 91 first slit
92 second slit
95, 96 first light shield
97 second light shield
100, 101 cell detector

**Claims**

1. A cell detector, comprising:

   a light emitter;
   a culture vessel configured to accommodate a culture medium containing a cell;
   a first slit member including a first slit and a first light shield; and
   at least one light receiver configured to receive light emitted from the light emitter and passing through the culture medium and the first slit in an order of the culture medium and the first slit,
   wherein the first light shield in the first slit member blocks light emitted from the light emitter and scattered by the cell in the culture vessel when the cell is at a position overlapping the first slit.

2. The cell detector according to claim 1, wherein the light emitter faces the at least one light receiver with the first slit member in between.

3. The cell detector according to claim 1 or claim 2, wherein
   the first slit and the at least one light receiver overlap each other in a plan view.

4. The cell detector according to any one of claims 1 to 3, wherein
   the first slit member includes a plurality of first slits.

5. The cell detector according to claim 4, wherein

   the at least one light receiver includes a plurality of light receivers in one-to-one correspondence with the plurality of first slits,
a dimension of each of the plurality of light receivers in a width direction and a dimension of each of the plurality of light receivers in a length direction orthogonal to the width direction are substantially equal to a dimension of a corresponding first slit of the plurality first slits in a width direction and a dimension of the first slit in a length direction orthogonal to the width direction,
each of the plurality of light receivers overlaps a corresponding first slit of the plurality first slits at substantially the same position in a plan view, and
each of the plurality of light receivers has an upper surface at a distance of 50 to 1,000 μm from a bottom surface of the first slit member.

6. The cell detector according to any one of claims 3 to 5, wherein

   a dimension of each of the plurality of first slits in a width direction is smaller than a diameter of the cell, and
   a dimension of each of the plurality of first slits in a length direction is larger than the diameter of the cell.

7. The cell detector according to any one of claims 3 to 6, wherein

   a dimension of each of the plurality of first slits in a width direction is 7 to 25 μm, and
   a dimension of each of the plurality of first slits in a length direction is 25 to 1,000 μm.

8. The cell detector according to any one of claims 1 to 7, wherein
   the first slit member includes a light-shielding material in a portion of an upper surface of the first slit member excluding the first slit.

9. The cell detector according to any one of claims 1 to 8, wherein
   the at least one light receiver is between a bottom surface of the first slit member and a bottom surface of the culture vessel.

10. The cell detector according to any one of claims 1 to 8, wherein
    the at least one light receiver is below a bottom surface of the first slit member and directly below the culture vessel.

11. The cell detector according to any one of claims 1 to 10, further comprising:
    a second slit member between the first slit member and the at least one light receiver, the second slit member including a second slit and a second light shield.

**12.** The cell detector according to claim 11, wherein

the second slit member has the same shape as the first slit member and is at substantially the same position as the first slit member in a width direction and a length direction in a plan view, and

the second slit member has an upper surface at a distance of 1 to 100 μm from a bottom surface of the first slit member.

**13.** The cell detector according to claim 11 or claim 12, wherein
the at least one light receiver is between a bottom surface of the second slit member and a bottom surface of the culture vessel.

**14.** The cell detector according to claim 11 or claim 12, wherein
the at least one light receiver is below a bottom surface of the second slit member and directly below the culture vessel.

**15.** The cell detector according to any one of claims 1 to 14, further comprising:
a determiner configured to determine that the cell has proliferated to a predetermined number or greater in response to an amount of light received by the at least one light receiver being smaller than or equal to a predetermined amount.

**16.** The cell detector according to any one of claims 1 to 15, wherein
the cell includes an induced pluripotent stem cell.

**17.** The cell detector according to any one of claims 1 to 16, wherein
the at least one light receiver includes a photodiode.

**18.** A cell detector, comprising:

a light emitter;
a culture vessel configured to accommodate a culture medium containing a cell;
a first slit member including a first slit; and
a light receiver configured to receive light emitted from the light emitter and passing through the culture medium and the first slit in an order of the culture medium and the first slit,
wherein the first slit member causes light emitted from the light emitter and scattered by the cell in the culture vessel to avoid reaching the light receiver.

**19.** A cell detection method implementable with the cell detector according to any one of claims 1 to 18, the method comprising:

placing the culture medium and the cell in the culture vessel;
culturing the cell;
illuminating the culture medium containing the cell with light emitted from the light emitter; and
obtaining an amount of light passing through the first slit member and received by the light receiver, or an amount of light passing through the first slit member and the second slit member and received by the light receiver.

FIG. 1

FIG. 2

100

| Light emitter | 10 |

| First slit member | 20 |
| First slit | 90 |
| First light shield | 95 |

| Light receiver | 30 |  | Determiner | 50 |

| Culture vessel | 40 |

FIG. 3

W

90

L

RL

60

# FIG. 4

FIG. 5

FIG. 6

```
        ┌─────────────┐
        │    Start     │
        └──────┬──────┘
               │
     ┌─────────┴─────────┐   A1
     │     Placement      │
     └─────────┬─────────┘
               │
     ┌─────────┴─────────┐   A2
     │      Culture       │
     └─────────┬─────────┘
               │
     ┌─────────┴─────────┐   A3
     │    Illumination    │
     └─────────┬─────────┘
               │
     ┌─────────┴─────────┐   A4
     │ Light amount obtainment │
     └─────────┬─────────┘
               │
     ┌─────────┴─────────┐   A5
     │   Determination    │
     └─────────┬─────────┘
               │
        ┌──────┴──────┐
        │     End      │
        └─────────────┘
```

## INTERNATIONAL SEARCH REPORT

| International application No. |
|---|
| PCT/JP2020/032726 |

A. CLASSIFICATION OF SUBJECT MATTER
Int.Cl. C12M1/34(2006.01)i, G01N21/17(2006.01)i, C12Q1/06(2006.01)i
FI: C12M1/34B, C12Q1/06, G01N21/17Z

According to International Patent Classification (IPC) or to both national classification and IPC

B. FIELDS SEARCHED

Minimum documentation searched (classification system followed by classification symbols)
Int.Cl. C12M1/34, G01N21/17, C12Q1/06

Documentation searched other than minimum documentation to the extent that such documents are included in the fields searched
Published examined utility model applications of Japan    1922-1996
Published unexamined utility model applications of Japan    1971-2020
Registered utility model specifications of Japan    1996-2020
Published registered utility model applications of Japan    1994-2020

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

C. DOCUMENTS CONSIDERED TO BE RELEVANT

| Category* | Citation of document, with indication, where appropriate, of the relevant passages | Relevant to claim No. |
|---|---|---|
| A | JP 2017-166910 A (TOSHIBA CORPORATION) 21 September 2017 (2017-09-21), claims, paragraphs [0059]-[0084], fig. 9 | 1-19 |
| A | JP 2019-106944 A (OLYMPUS CORPORATION) 04 July 2019 (2019-07-04), claims, fig. 9 | 1-19 |
| A | JP 2018-117557 A (PANASONIC INTELLECTUAL PROPERTY MANAGEMENT CO., LTD.) 02 August 2018 (2018-08-02), claims, fig. 7 | 1-19 |

☐ Further documents are listed in the continuation of Box C.     ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 29 September 2020 | 02 November 2020 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

| INTERNATIONAL SEARCH REPORT | International application No. |
| Information on patent family members | PCT/JP2020/032726 |

| | | |
|---|---|---|
| JP 2017-166910 A | 21 September 2017 | US 2017/0268982 A1<br>claims, paragraphs [0090]-[0115]<br>fig. 9 |
| JP 2019-106944 A | 04 July 2019 | (Family: none) |
| JP 2018-117557 A | 02 August 2018 | US 2018/0210185 A1<br>claims, fig. 7 |

Form PCT/ISA/210 (patent family annex) (January 2015)

**EP 4 036 210 A1**

**REFERENCES CITED IN THE DESCRIPTION**

**Patent documents cited in the description**

- JP 2019106944 A
- JP 2018515787 W
- WO 2016142043 A
- JP 2015528311 W